# FASCICULE DE BREVET EUROPEEN

(11) **EP 1 523 936 B1**
(45) Date de publication et mention de la délivrance du brevet: **25.11.2009**
(21) Numéro de dépôt: 04292458.9
(22) Date de dépôt: 15.10.2004
(51) Int. Cl.: A61B 5/103

(54) **Système permettant d'analyser la peau**
System für die Analyse der Haut
System for skin analysis

(30) Priorité: 16.10.2003 FR 0312105
(43) Date de publication de la demande: 20.04.2005
(73) Titulaire: L'ORÉAL, 75008 Paris (FR)
(72) Inventeur: Bazin, Roland, 91570 Bièvres (FR)
(74) Mandataire: Tanty, François

(56) Documents cités:
- EP-A- 1 277 436
- EP-A- 1 314 395

## Description

La présente invention est relative notamment à un système permettant d'analyser la peau d'un être humain.

L'expérience montre que souvent les consommateurs ne choisissent pas de manière optimale les produits de maquillage et/ou de soin de la peau, parce qu'ils ne connaissent pas toujours précisément leur type de peau et ses besoins spécifiques.

La demande EP 1 314 395 décrit un procédé pour connaître l'effet de l'environnement sur une portion de la peau et la demande EP 1 277 436 décrit un dispositif pour observer la peau et les cheveux.

Il existe encore un besoin pour permettre d'analyser la peau de manière satisfaisante afin notamment de fournir aux consommateurs des conseils personnalisés.

L'invention a ainsi pour objet un système permettant d'analyser la peau, comportant :
- une caméra pour acquérir au moins une image de la peau avec un grossissement permettant d'observer les pores et/ou les taches de la peau,
- des moyens d'affichage permettant d'afficher simultanément au moins une image de la peau ainsi acquise et une image d'une peau de référence, ces images peuvent notamment correspondre à une même portion du corps humain,
- un appareil autre qu'une caméra pour mesurer une caractéristique physico-chimique de la peau,
- des moyens de traitement pour délivrer une information concernant l'état de la peau à partir du traitement d'au moins une image acquise et/ou d'une mesure effectuée par l'appareil.

Par « caractéristique physico-chimique », on entend notamment la carnation complexion de la peau, la concentration de sébum de la peau, le degré d'hydratation de la peau ou le degré de fermeté de la peau.

Grâce à l'invention, il est possible de connaître précisément l'état de la peau du sujet et donc de déterminer ses besoins spécifiques, du fait que l'on réalise une analyse de la peau à partir non seulement d'au moins une image de cette peau que l'on peut comparer à une image de référence mais également à partir d'au moins une mesure non optique.

Cela peut permettre, notamment sur un point de vente ou dans un salon de beauté, de conseiller précisément le consommateur dans le choix d'un produit de maquillage et/ou de soin adapté à sa peau.

De préférence, la caméra comporte un objectif grossissant plus de 40 fois, notamment 60 fois. Un tel grossissement s'avère adapté à un traitement de l'image en vue par exemple de quantifier le nombre de pores ou de taches de la peau.

Les moyens de traitement sont avantageusement agencés pour déterminer à partir d'au moins une image de la peau acquise par la caméra au moins l'un du nombre, notamment du nombre moyen, de la taille, notamment de la taille moyenne, et de la localisation des pores et/ou des taches de la peau.

Dans un exemple de mise en oeuvre de l'invention, la caméra comporte un polariseur permettant une observation sous la surface de la peau.

Le système comporte un appareil pour collecter du sébum sur la peau, par exemple pour la zone U et/ou la zone T du visage, cet appareil comportant un support de collecte de sébum constitué par une pastille, avec une face d'application sur la peau. Les moyens de traitement sont agencés pour déterminer la quantité de sébum collectée à partir du traitement d'une image de ce support de collecte.

Le système peut comporter en plus de la première caméra permettant l'observation des pores et/ou des taches de la peau, une deuxième caméra comportant un objectif de plus faible grossissement, notamment d'un facteur de grossissement inférieur à 20, par exemple égal à 10, pour acquérir au moins une image de la peau permettant d'analyser la profondeur des rides de celle-ci.

Les moyens de traitement peuvent notamment être agencés pour déterminer un degré de rugosité de la peau à partir d'une image des rides de la peau acquise avec une telle caméra.

Le système peut en outre comporter un élastomètre pour déterminer un degré de fermeté de la peau et/ou un coméomètre pour déterminer un degré d'hydratation de la peau.

De préférence, les moyens de traitement sont agencés pour permettre la comparaison des données obtenues pour un sujet à des données de référence.

Ces données de référence peuvent notamment être obtenues à partir de mesures sur un groupe d'individus considérés comme ayant une peau idéale, et être associées à des tranches d'âge respectives et à un type de population donné. La peau servant de référence peut être ou non une peau idéale.

Pour déterminer une peau idéale pour une tranche d'âge donnée et une population donnée, il est possible par exemple d'évaluer au moins une caractéristique de la peau pour chaque personne de cette population en attribuant à chaque personne un score choisi sur une échelle donnée. Une peau idéale peut correspondre par exemple à une peau ayant le meilleur score pour cette population ou une peau ayant un score tombant dans un percentile prédéterminé, par exemple dans les dix meilleurs percentiles.

Par exemple, si la caractéristique de la peau est l'intensité des rides, le meilleur score et la peau idéale peuvent correspondre à la peau la moins ridée pour la population étudiée et une tranche d'âge donnée.

Une peau idéale peut encore être considérée comme une peau ayant un score meilleur que le score moyen ou le score médian déterminé pour une tranche d'âge donnée, une population donnée et une caractéristique de peau donnée.

Une peau idéale peut aussi être la peau d'individus célèbres, connus pour leur beauté, par exemple des modèles, acteurs ou actrices. Une peau idéale peut résulter par exemple d'une sélection dans un panel de différentes peaux, la sélection étant réalisée par exemple sur la base de considérations esthétiques.

Dans un exemple de mise en oeuvre de l'invention, le système est agencé pour permettre d'entrer des informations personnelles concernant le sujet examiné, ces informations pouvant comprendre des indications par exemple sur l'une au moins de la perception qu'a le sujet de l'état de sa peau, ses préoccupations concernant sa peau et ses habitudes en matière de beauté.

Le système peut être agencé pour fournir un questionnaire, notamment sous forme électronique, permettant d'obtenir, en réponse, lesdites informations.

Le système peut être agencé pour délivrer, par exemple sous la forme d'une page affichée par les moyens d'affichage, une représentation graphique synthétique, notamment de type histogramme, présentant des données obtenues pour un sujet en regard de données de référence.

Dans un exemple de mise en oeuvre de l'invention, le système comporte un espace de stockage de données, permettant de mémoriser les données obtenues pour un sujet.

L'invention a encore pour objet, selon un autre de ses aspects, un procédé pour délivrer un conseil en matière de beauté, caractérisé par le fait qu'il comporte les étapes suivantes :
- acquérir une image de la peau d'un sujet permettant d'observer les pores et/ou les taches de la peau, cette dernière pouvant avoir subi ou non préalablement un traitement cosmétique et/ou de soin,
- afficher l'image acquise simultanément avec une image d'une peau de référence,
- mesurer une caractéristique physico-chimique de la peau à l'aide d'un appareil autre qu'une caméra,
- délivrer un conseil en matière de beauté à partir du traitement de l'image acquise et/ou d'une mesure effectuée par l'appareil.

De préférence, on détermine l'un au moins de :
- le nombre, la taille et la localisation des pores de la peau,
- le nombre, la taille et la localisation des taches de la peau,
- la complexion de la peau,
- la profondeur des rides de la peau,
- la quantité de sébum pour au moins une zone de la peau,
- le degré d'hydratation de la peau,
- le degré de fermeté de la peau.

Si on le souhaite, on détermine toutes les caractéristiques précitées.

Dans un exemple de mise en oeuvre de l'invention, on compare les données déterminées pour le sujet à des données de référence associées à une tranche d'âge sélectionnée, cette tranche d'âge pouvant correspondre ou non à l'âge du sujet. Il est ainsi possible de conclure par exemple sur le fait que le sujet présente des caractéristiques de peau correspondant à celles d'une tranche d'âge autre que la sienne.

Le procédé peut notamment comporter l'étape consistant à obtenir des informations personnelles concernant le sujet, ces informations contenant par exemple des indications sur la perception qu'a le sujet de l'état de sa peau.

Dans un exemple de mise en oeuvre de l'invention, on compare le résultat de l'analyse aux informations fournies par le sujet sur la perception qu'a le sujet de sa peau.

Le procédé peut comporter l'étape consistant à prescrire un ou plusieurs produits de soin ou un ou plusieurs compléments nutritionnels en fonction des résultats de l'analyse et des informations fournies par le sujet.

L'invention a encore pour objet, selon un autre de ses aspects, un procédé comportant les étapes suivantes :
- acquérir au moins une image de la peau d'un sujet,
- mesurer au moins une caractéristique physico-chimique de la peau d'un sujet à l'aide d'un appareil autre qu'une caméra,
- permettre au sujet de fournir des informations concernant sa perception de sa peau,
- comparer au moins une information résultant du traitement de ladite au moins une image et/ou de ladite au moins une caractéristique mesurée avec les informations fournies par le sujet.

Le procédé peut comporter l'étape consistant à délivrer un conseil en matière de beauté ou une recommandation d'un produit, sur la base de la comparaison précitée.

L'invention a encore pour objet, selon un autre de ses aspects, un système pour analyser la peau d'un sujet, le système comportant :
- une première caméra pour acquérir au moins une image de la peau avec un grossissement permettant d'observer les pores et/ou les taches de la peau,
- une deuxième caméra comportant un objectif de plus faible grossissement pour acquérir au moins une image de la peau permettant d'analyser la profondeur des rides,
- un élastomètre pour déterminer un degré de fermeté de la peau,
- un cornéomètre pour déterminer un degré d'hydratation de la peau,
- un appareil pour collecter du sébum sur la peau,
- des moyens de traitement pour délivrer une information concernant la peau par traitement de ladite au moins une image acquise et/ou d'une mesure réalisée par un des appareils ci-dessus.

L'invention a encore pour objet, selon un autre de ses aspects, un procédé comportant les étapes suivantes :
- mesurer au moins une caractéristique physico-chimique de la peau en utilisant un appareil autre qu'une caméra,
- comparer ladite au moins une caractéristique mesurée avec des données de référence associées à au moins un individu considéré comme ayant une peau idéale.

L'invention pourra être mieux comprise à la lecture de la description détaillée qui va suivre, d'un exemple de mise en oeuvre non limitatif de l'invention, et à l'examen du dessin annexé, sur lequel :
- la figure 1 représente, schématiquement et partiellement, un système d'analyse conforme à l'invention,
- la figure 2 représente, schématiquement et partiellement, un appareil pour déterminer la quantité de sébum d'une zone de la peau,
- la figure 3 illustre, schématiquement et partiellement, l'utilisation de l'appareil de la figure 2,
- la figure 4 illustre, schématiquement et partiellement, l'acquisition d'une image de la face d'application du support de collecte de sébum,
- la figure 5 est un schéma en blocs illustrant différentes étapes d'un procédé pour délivrer un conseil en matière de beauté, et

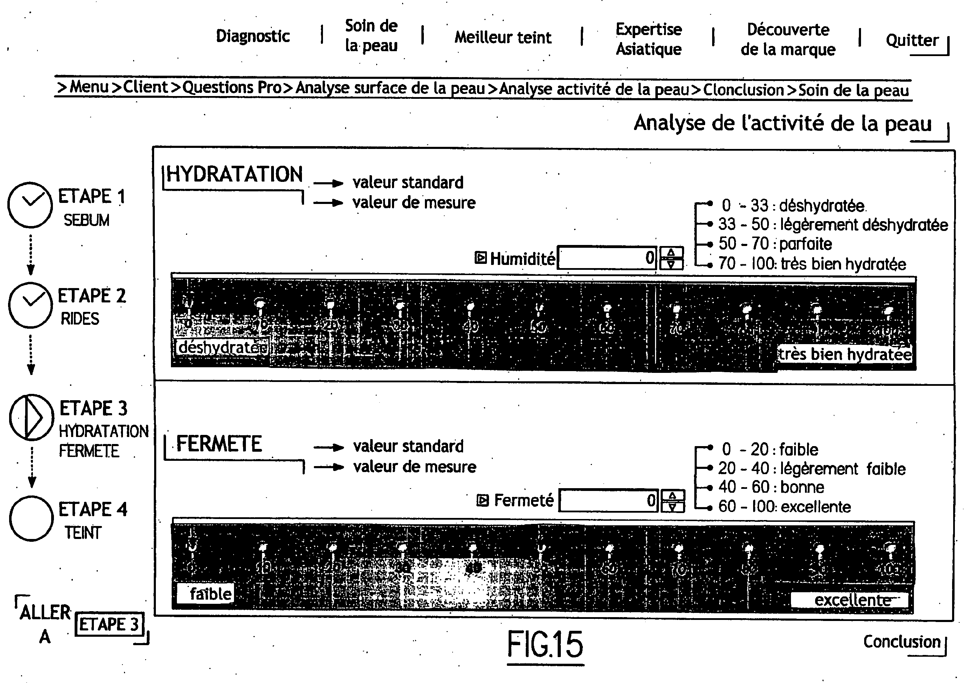

- les figures 6 à 18 représentent, schématiquement et partiellement, des pages écran affichées par le système de la figure 1.

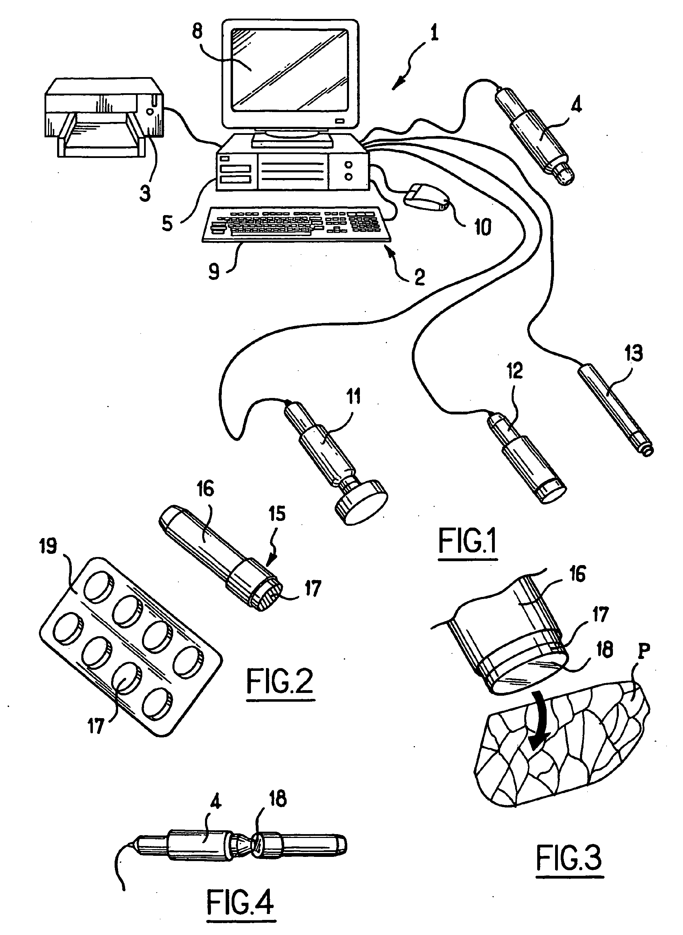

On a représenté sur la figure 1 un système 1 permettant d'analyser la peau P d'un sujet, comportant un ordinateur 2, une imprimante 3 reliée à l'ordinateur 2 et une première caméra 4, également reliée à l'ordinateur 2.

L'ordinateur 2 qui peut être conventionnel comporte une unité centrale 5, un écran 8 et une interface utilisateur comportant un clavier 9 et une souris 10.

La première caméra 4 comporte un objectif grossissant soixante fois et elle est équipée d'un polariseur non apparent, d'orientation réglable et comporte un éclairage intégré.

Le système 1 comporte en outre, relié à l'ordinateur 2 :
- une deuxième caméra 11 avec un objectif grossissant dix fois,
- un élastomètre 12,
- un coméomètre 13.

Le système d'analyse 1 comporte un appareil 15, représenté sur la figure 2, permettant de collecter du sébum sur la peau d'un sujet.

L'appareil 15 est par exemple un sébumètre.

Cet appareil 15 comporte un organe de préhension 16 dont une extrémité est agencée pour recevoir une pastille 17 de collecte de sébum. Cette pastille 17 est dans l'exemple considéré conditionnée avant utilisation dans une plaquette 19.

L'appareil 15 s'utilise de la manière suivante.

On fixe une pastille de collecte de sébum 17 sur l'organe de préhension 16, puis on applique une face 18 de la pastille 17 sur la peau P du sujet. Après enlèvement de la pastille, on acquiert à l'aide de la caméra 4, comme illustré sur la figure 4, une image de la face 18 de la pastille 17 ayant collecté du sébum.

Un traitement de cette image permet de déterminer la quantité de sébum collectée.

Dans l'exemple considéré, le système 1 est en outre agencé pour permettre d'entrer des informations personnelles concernant le sujet.

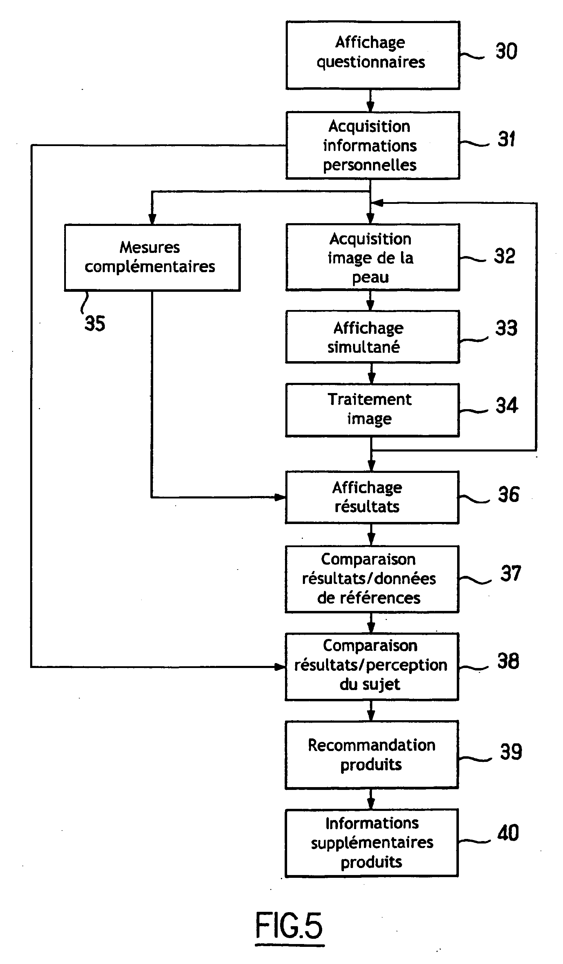

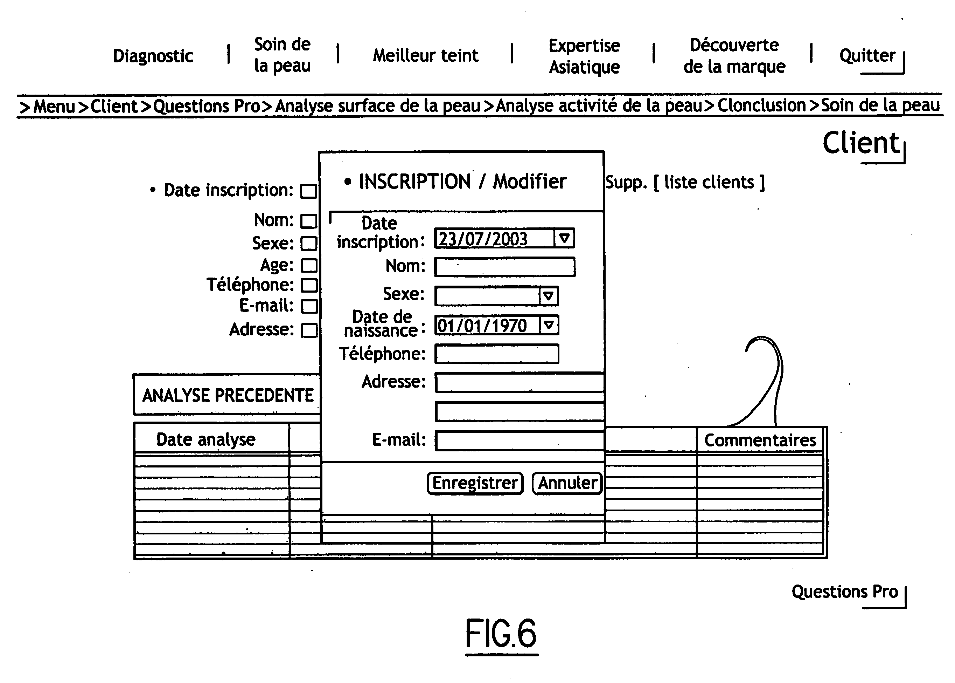

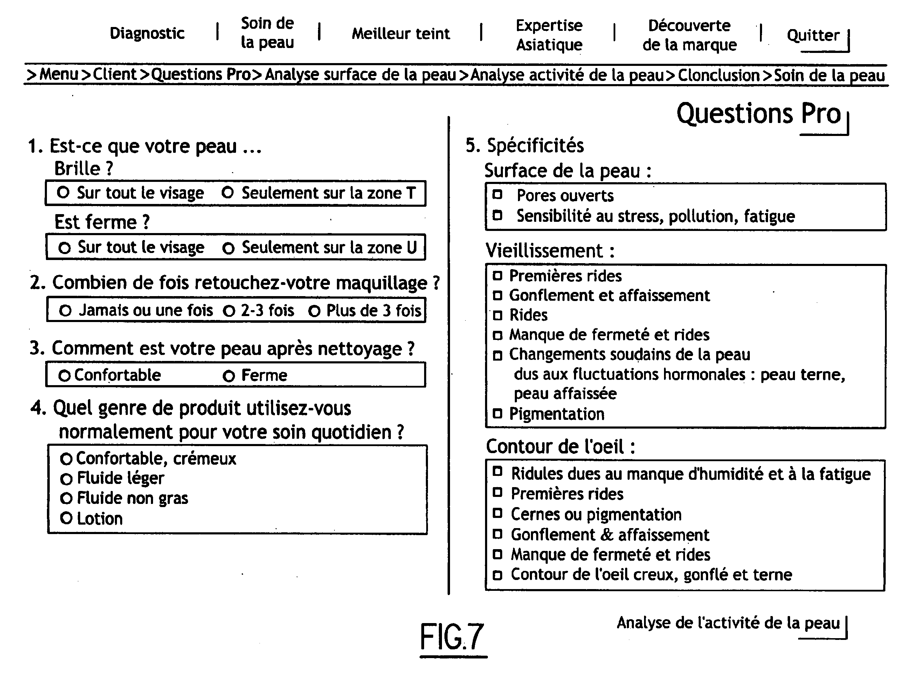

Pour ce faire, l'ordinateur 2 procède, comme illustré à la figure 5, dans une première étape 30, à l'affichage sur l'écran 8 de questionnaires tels que ceux illustrés respectivement sur les figures 6 et 7.

Le questionnaire représenté sur la figure 6 permet d'entrer à l'étape 31 des informations telles que la date d'inscription, le nom, le sexe, la date de naissance, le numéro de téléphone, l'adresse postale et l'adresse de courrier électronique du sujet.

Ces informations sont par exemple mémorisées dans l'unité centrale 5.

Le questionnaire représenté sur la figure 7 comporte une pluralité de questions concernant la perception qu'a le sujet de sa peau, ses habitudes de maquillage et ses attentes en matière de beauté.

Ces questions, données à titre d'exemples, sont par exemple du type :
- votre peau brille t-elle ?
- votre peau semble-t-elle ferme ?
- combien de fois retouchez-vous votre maquillage : jamais ou une fois, deux-trois fois, plus de trois fois ?
- après nettoyage, votre peau semble-t-elle : confortable ? ferme ?

A l'étape 32, le sujet lui-même ou une esthéticienne le cas échéant, procède à l'acquisition d'une image 40 de la peau à l'aide de la première caméra 4.

Le grossissement de la première caméra 4 permet d'observer les pores et les taches de la peau.

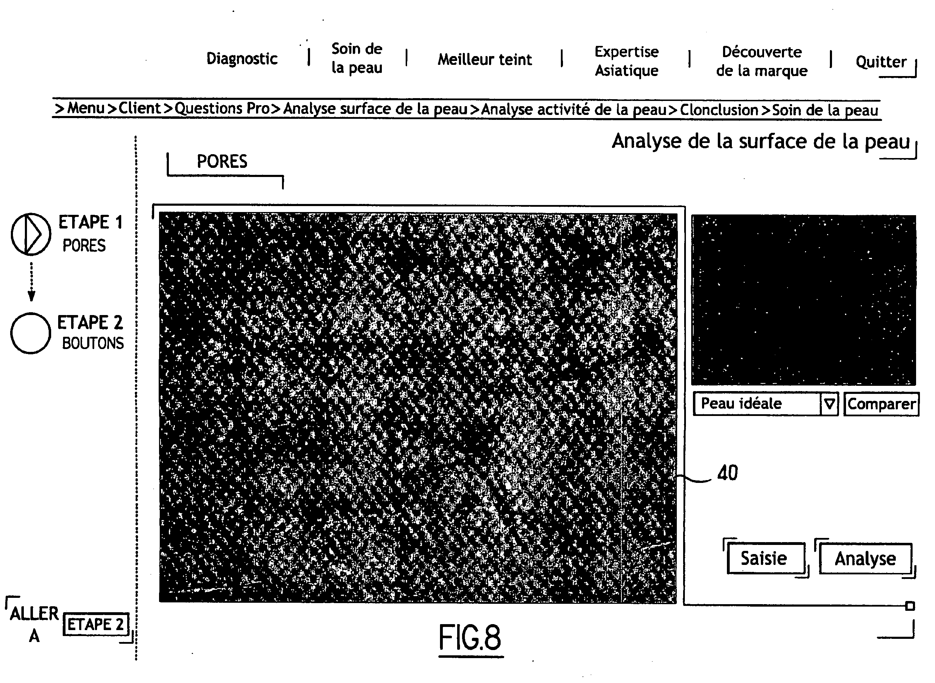

L'image 40 est affichée sur l'écran 8, comme on peut le voir sur la figure 8.

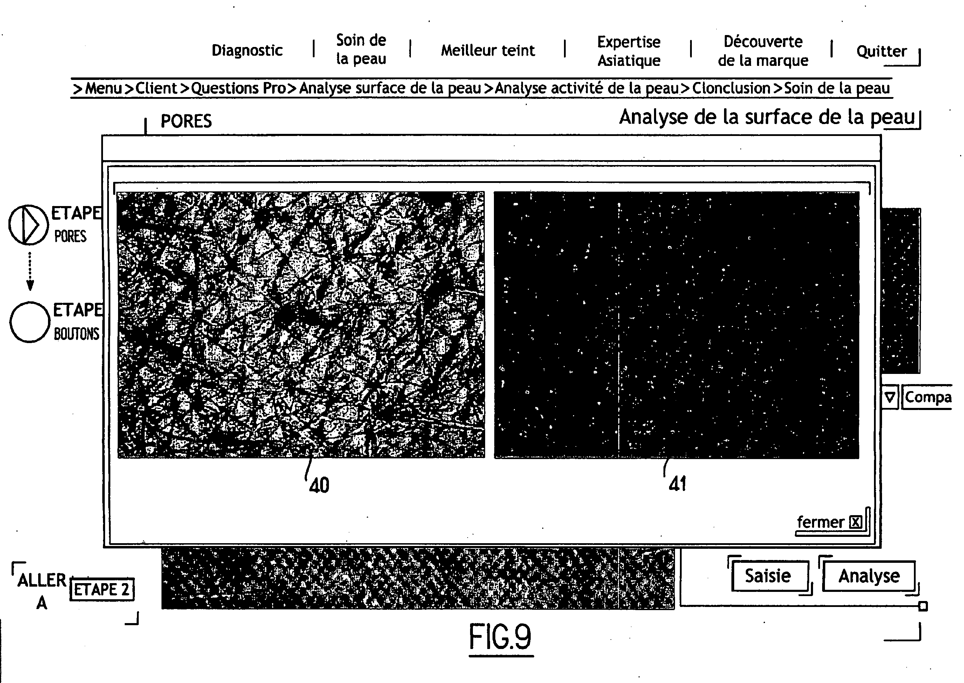

A l'étape 33, on procède à l'affichage simultané de l'image 40 de la peau du sujet et d'une image 41 d'une peau de référence, ces images ayant le même formant, comme illustré sur la figure 9.

Dans l'exemple considéré, les peaux de référence sont associées à des tranches d'âges respectives et sont chacune obtenues à partir de la peau de cinq personnes d'une tranche d'âge donnée et appartenant à un type de population donné, considérées comme idéales.

On peut ainsi comparer l'image de la peau du sujet avec une image d'une peau de référence de la tranche d'âge du sujet et du type de population du sujet.

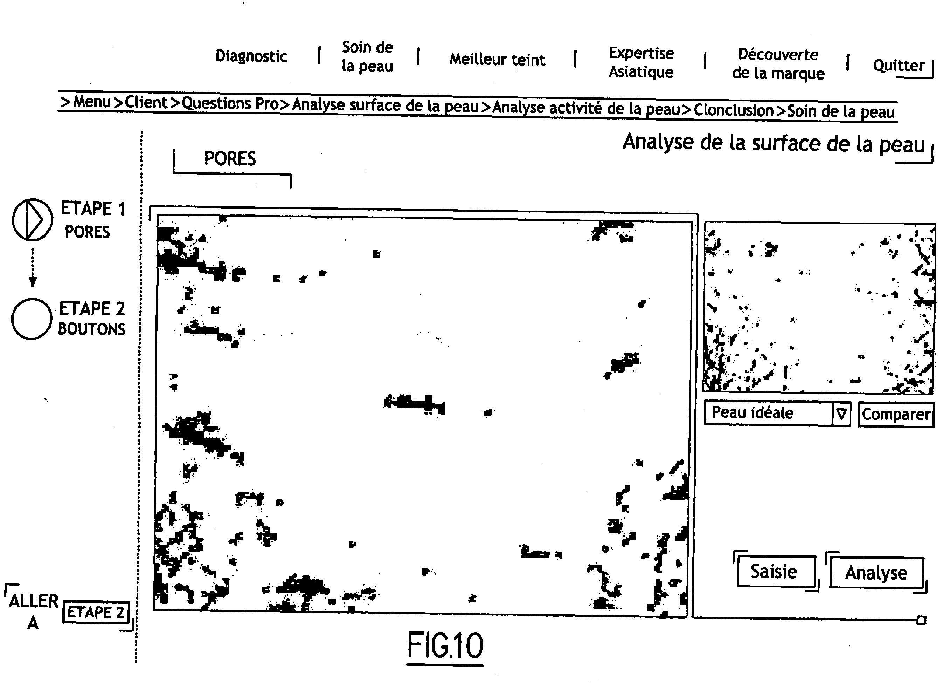

A l'étape suivante 34, l'image 40 est traitée par détection binaire comme illustré sur la figure 10, afin de déterminer notamment le nombre, la taille et la localisation des pores de la peau.

Il est possible de répéter les étapes 32 à 34 en observant les taches de la peau.

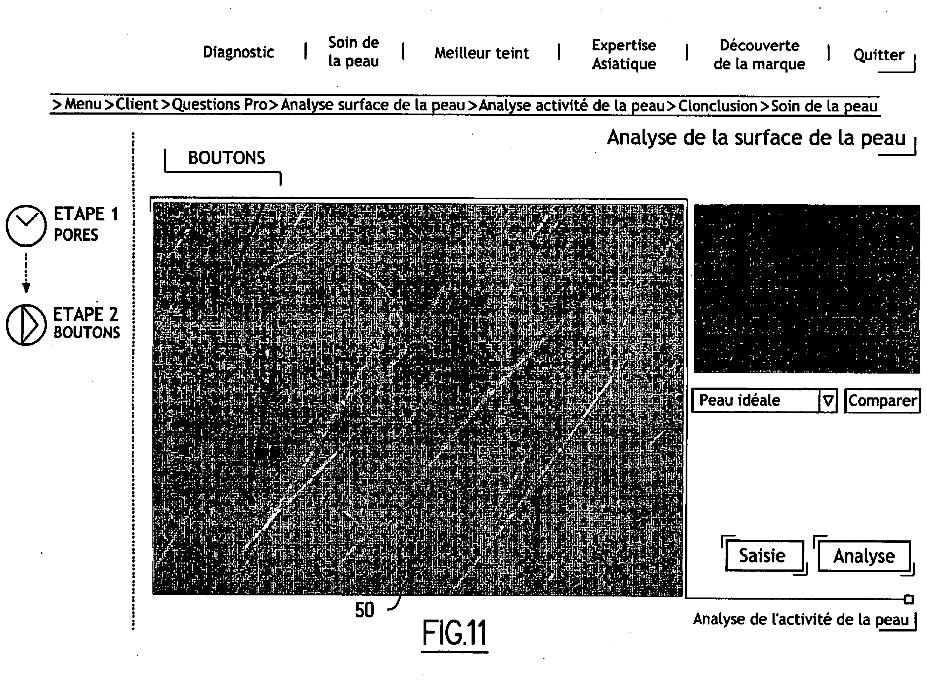

Dans ce cas, à l'étape 32, une image 50 de la peau, représentée sur la figure 11, est acquise en utilisant la caméra 4 équipée du polariseur.

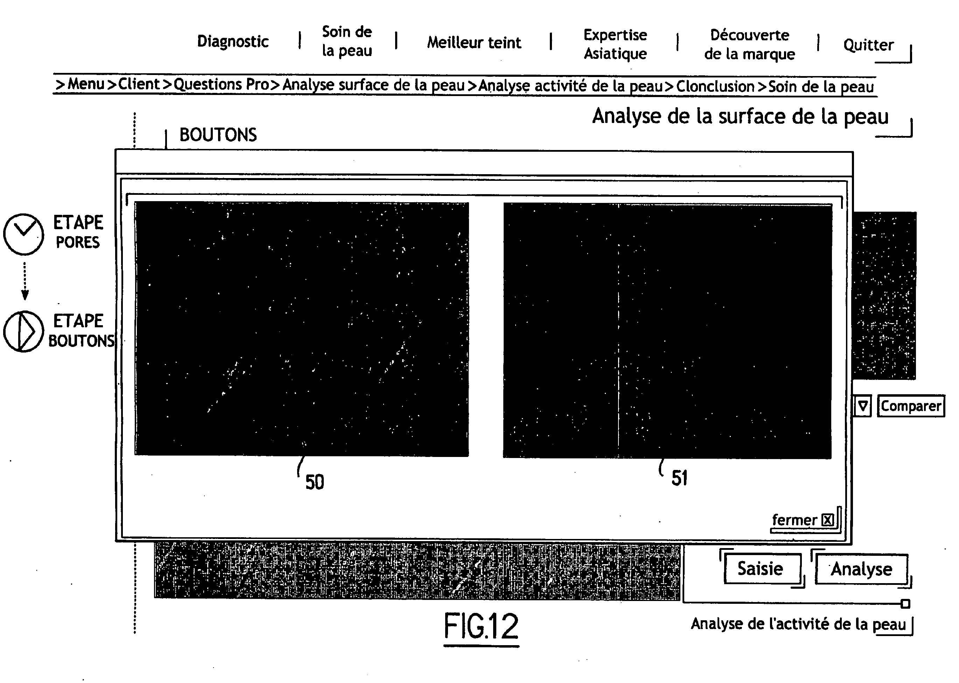

A l'étape 33, on peut afficher l'image 50 simultanément avec une image 51 d'une peau de référence en vue de les comparer, comme illustré à la figure 12.

A l'étape 34, avec la détection binaire, il est possible de déterminer notamment le nombre, la taille et la localisation des taches de la peau.

Afin d'affiner l'analyse de la peau, on peut procéder à des mesures complémentaires sur la peau à l'étape 35.

On peut notamment mesurer la quantité de sébum de la peau du sujet.

A cette fin, on utilise l'appareil 15 permettant de collecter du sébum sur une pastille 17.

A l'aide de la caméra 4, on procède à l'acquisition d'une image de la face d'application de la pastille 17.

Cette opération est effectuée respectivement pour la zone U du visage et la zone T du visage. La zone U correspond à la région du visage s'étendant d'une joue à l'autre en passant par le menton. La zone T correspond à la région du visage définie par le front et le nez.

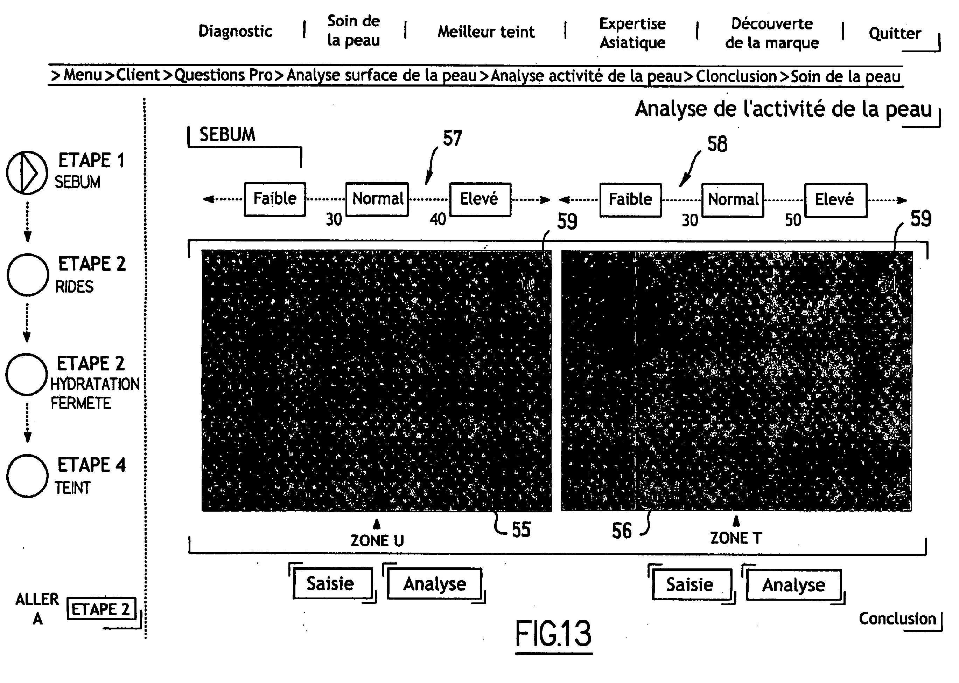

On a représenté sur la figure 13 deux images 55 et 56 obtenues respectivement pour la zone U et la zone T. A partir de ces images 55 et 56, l'unité centrale 5 calcule la quantité de sébum pour chaque zone.

Les valeurs obtenues 59 sont comparées à des valeurs de référence respectives, présentées par exemple sur des échelles 57 et 58, ce qui permet de conclure quant à la présence de sébum en défaut ou en excès.

Par exemple, dans la zone U, la valeur mesurée correspond à une situation en défaut de sébum. En revanche, la valeur mesurée pour la zone T correspond à une situation en excès de la quantité de sébum.

Pour déterminer la rugosité de la peau, on procède à l'acquisition d'une image 60 à l'aide de la caméra 11.

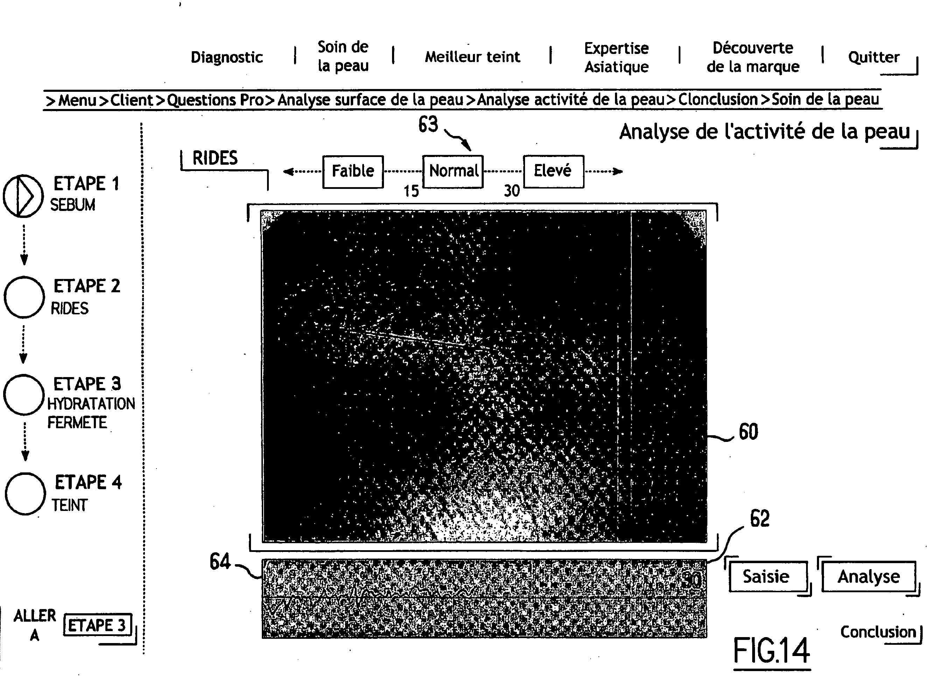

L'image acquise 60 est affichée, comme illustré sur la figure 14, et permet par exemple de distinguer des rides autour de l'oeil.

L'unité centrale 5 est agencée pour déterminer, à partir de cette image 60, pour une ligne 61 sélectionnée, un degré de rugosité 62 de la peau par évaluation des variations du relief de la peau le long de cette ligne 61, comme représenté sur le graphe 64.

Ce degré mesuré peut être comparé à des valeurs de référence 63, affichées au-dessus de l'image 60.

Comme on peut le voir, le degré mesuré dans l'exemple décrit correspond à une situation normale.

On détermine ensuite le degré de fermeté de la peau au moyen de l'élastomètre 12 et le degré d'hydratation de la peau grâce au cornéomètre 13.

Les valeurs mesurées d'hydratation et de fermeté peuvent être comparées respectivement à des échelles d'hydratation et de fermeté, comme illustré sur la figure 14.

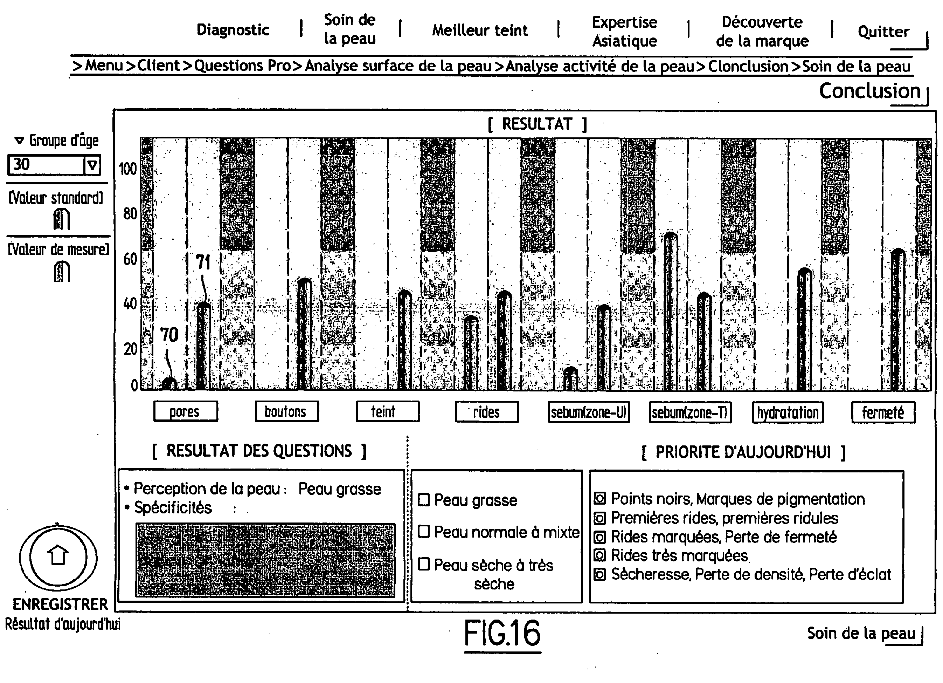

Après avoir effectué ces mesures, à l'étape 36, on procède à l'affichage de l'ensemble des résultats obtenus en regard de valeurs de référence, sous la forme d'une représentation graphique de type histogramme, comme on peut le voir sur la figure 16.

On obtient ainsi une vue synthétique des résultats.

La représentation graphique des résultats peut par exemple être imprimée sur papier grâce à l'imprimante 3, ou être mémorisée par l'unité centrale 5.

Ces résultats peuvent également être envoyés via un réseau téléphonique ou informatique, notamment Internet, par exemple vers le terminal portable ou l'ordinateur personnel du sujet.

Des colonnes 70 correspondant aux valeurs mesurées pour la peau du sujet sont mises côte à côte avec des colonnes 71 correspondant à des valeurs de référence, pour respectivement chaque caractéristique de la peau, à savoir par exemple le nombre de pores, de taches, la complexion, les rides, la quantité de sébum, respectivement pour la zone U et pour la zone T, l'hydratation et la fermeté.

Les valeurs de référence sont associées à des tranches d'âge respectives et sont obtenues comme pour les images des peaux de référence, à partir de mesures sur des groupes d'individus considérés comme ayant une peau idéale.

A l'étape 37, on compare les résultats de l'analyse aux données de référence, notamment celles associées à une tranche d'âge particulière, en vue de déterminer les défauts et les qualités de la peau du sujet.

L'esthéticienne peut par exemple conclure que, le sujet ayant 40 ans, l'hydratation de sa peau correspond à celle d'une personne de 30 ans.

Inversement, l'esthéticienne peut estimer que, le sujet ayant 30 ans, la fermeté de sa peau correspond à celle d'une personne de 40 ans.

Sous l'histogramme, figurent à gauche les informations données par le sujet concernant notamment la perception qu'il a de sa peau, et à droite les conclusions déduites des mesures, permettant leur comparaison à l'étape 38.

L'esthéticienne, au vu, d'une part, des informations concernant la perception qu'a le sujet de sa peau et, d'autre part, des résultats des mesures, peut conseiller à l'étape 39 le consommateur dans le choix de produits de maquillage, de soin ou de traitement, le cas échéant de compléments nutritionnels.

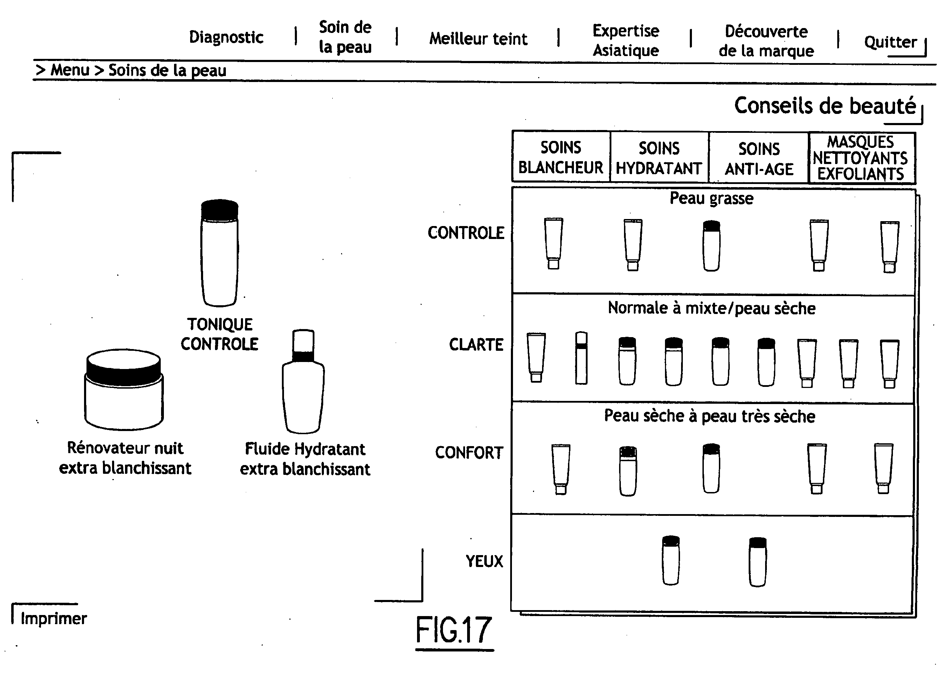

Comme on peut le voir sur la figure 17, il est possible de recommander parmi un ensemble de produits cosmétiques, ceux qui sont le mieux adaptés au sujet.

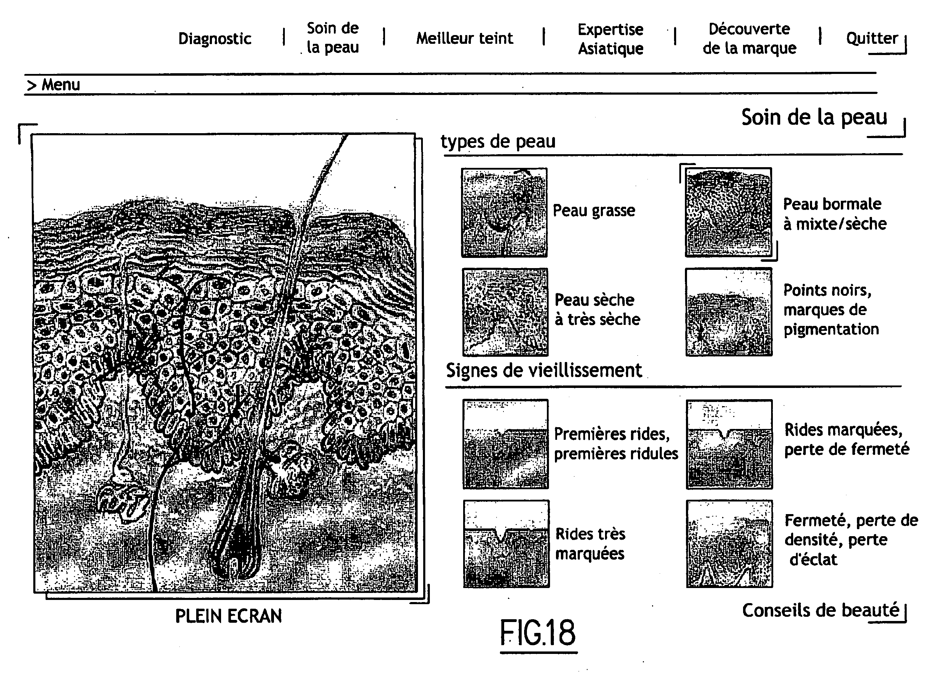

L'esthéticienne peut enfin, à l'aide de pages écran telles que celle représentée sur la figure 18, fournir des explications au sujet concernant l'état de sa peau.

Dans toute la description, y compris les revendications, l'expression « comportant un » doit être comprise comme étant synonyme de « comportant au moins un », sauf si le contraire est spécifié.

L'invention n'est pas limitée à l'exemple qui vient d'être décrit.

Le système d'analyse peut notamment comporter d'autres appareils de mesure permettant d'évaluer l'état de la peau.

## Revendications

1. Système (1) permettant d'analyser la peau d'un sujet, comportant :
- une caméra (4) pour acquérir au moins une image de la peau avec un grossissement permettant d'observer les pores et/ou les taches de la peau.
- des moyens d'affichage (8) pour afficher simultanément au moins une image acquise et une image d'une peau de référence,
- un appareil (12; 13 ; 15) autre qu'une caméra pour mesurer une caractéristique pbysico-chimique de la peau,
- des moyens de traitement (5) pour délivrer une information concernant l'état de la peau à partir du traitement d'au moins une image acquise et/ou d'une mesure effectuée par l'appareil,
- un appareil (15) pour collecter du sébum sur la peau, comportant un support de collecte de sébum **caractérisé en ce qu'**il comporte une pastille (17) ayant une face d'application sur la peau, et que les moyens de traitement (5) sont agencés pour déterminer la quantité de sébum collectée à partir du traitement d'une image du support de collecte (17).

2. Système selon la revendication précédente, **caractérisé par le fait que** la caméra (4) comporte un objectif grossissant plus de 40 fois, par exemple 60 fois.

3. Système selon l'une des revendications 1 et 2, **caractérisé par le fait que** les moyens de traitement (5) sont agencés pour déterminer à partir d'au moins une image de la peau acquise par la caméra au moins l'un du nombre, de la taille et de la localisation des pores de la peau.

4. Système selon l'une quelconque des revendications précédentes, **caractérisé par le fait que** les moyens de traitement (5) sont agencés pour déterminer, à partir d'au moins une image de la peau acquise par la caméra, au moins l'un du nombre, de la taille et de la localisation des taches de la peau.

5. Système selon l'une quelconque des revendications précédentes, **caractérisé par le fait que** la caméra (4) comporte un polariseur.

6. Système selon l'une quelconque des revendications précédentes, **caractérisé par le fait qu'**il comporte une deuxième caméra (11) comportant un objectif de plus faible grossissement que celui de la première caméra, notamment d'un facteur de grossissement égal à 10, pour acquérir au moins une image de la peau permettant d'analyser les rides de la peau.

7. Système selon la revendication précédente, **caractérisé par le fait que** les moyens de traitement (5) sont agencés pour déterminer un degré de rugosité de la peau à partir d'une image des rides de la peau.

8. Système selon l'une quelconque des revendications précédentes, **caractérisé par le fait qu'**il comporte un élastomètre (12) pour déterminer un degré de fermeté de la peau.

9. Système selon l'une quelconque des revendications précédentes, **caractérisé par le fait qu'**il comporte un cornéomètre (13) pour déterminer un degré d'hydratation de la peau.

10. Système selon l'une quelconque des revendications précédentes, **caractérisé par le fait que** les moyens de traitement (5) sont agencés pour permettre la comparaison des données résultant de l'analyse à des données de référence.

11. Système selon la revendication précédente, **caractérisé par le fait que** les données de référence sont obtenues à partir de mesures sur un groupe d'individus considérés comme ayant une peau idéale, les données de référence étant associées à des tranches d'âge respectives et à un type de population donné.

12. Système selon l'une quelconque des revendications précédentes, **caractérisé par le fait qu'**il est agencé pour permettre d'entrer des informations personnelles du sujet.

13. Système selon la revendication 12, **caractérisé par le fait qu'**il est agencé pour fournir un questionnaire, notamment sous forme électronique, permettant d'obtenir, en réponse, lesdites informations.

14. Système selon l'une quelconque des revendications précédentes, **caractérisé par le fait qu'**il est agencé pour délivrer, par exemple sous la forme d'une page affichés par les moyens d'affichage, une représentation graphique notamment de type histogramme, présentant le résultat de l'analyse en regard de données de référence.

15. Système selon l'une quelconque des revendications précédentes, **caractérisé par le fait qu'**il comporte un espace de stockage de données pour mémoriser le résultat de l'analyse.

16. Système selon l'une quelconque des revendications précédentes, l'appareil (15) pour collecter du sébum sur la peau comportant un organe de préhension (16) dont une extrémité est agencée pour recevoir la pastille (17).

17. Système selon l'une quelconque des revendications précédentes, la pastille (17) étant conditionnée avant utilisation dans une plaquette (19).

18. Système selon l'une des revendications précédentes, l'appareil (15) pour collecter du sébum sur la peau étant un sébumètre.

19. Système selon la revendication 1, l'image du support de collecte ayant été acquise à l'aide de la caméra (4).

## Claims

1. A system (1) for analyzing the skin of a subject, the system comprising:
a camera (4) for acquiring at least one image of the skin at a magnification that enables the pores and/or the spots of the skin to be observed;
■ a display device (8) for displaying simultaneously at least one acquired image and one image of a reference skin;
■ an apparatus (12; 13; 15) other than a camera for measuring at least one physico-chemical characteristic of the skin;
■ a processor (5) for delivering information relating to the skin by processing at least one acquired image and/or a measurement performed by the apparatus,
■ a apparatus (15) for collecting sebum on the skin comprising a sebum-collector medium
wherein the sebum-collector medium comprises a pellet (17) having a face for application against the skin, and the processor (5) is configured to determine the quantity of collected sebum by processing at least one image of the collector medium (17).

2. A system according to preceding claim, wherein the camera (4) has an objective lens with at least 40 times magnification, e.g. 60 times magnification.

3. A system according to any of claim 1 and 2, wherein the processor (5) is configured to determine from at least one image of the skin acquired by the camera at least one of the following: the number, the size, and the locations of pores in the skin.

4. A system according to any preceding claim, wherein the processor (5) is configured to determine, from at least one image of the skin acquired by the camera, at least one of the following: the number, the size, and the locations of spots on the skin.

5. A system according to any preceding claim, wherein the camera (4) comprises a polarizer.

6. A system according to any preceding claim, wherein the system comprises a second camera (11) comprising an objective lens of lower magnification than that of the first camera, e.g. of magnification equal to 10, in order to acquire at least one image of the skin enabling the wrinkles of the skin to be analyzed.

7. A system according to preceding claim, wherein the processor (5) is configured to determine a degree of roughness of the skin from an image of wrinkles of the skin.

8. A system according to any preceding claim, wherein the system comprises an elasticity meter (12) for determining a degree of firmness of the skin.

9. A system according to any preceding claim, wherein the system comprises a corneometer (13) for determining a degree of hydration of the skin.

10. A system according to any preceding claim, wherein the processor (5) is configured to enable the data that results from the analysis to be compared with reference data.

11. A system according to preceding claim, wherein the reference data is obtained from measurements taken on a group of individuals considered as having ideal skin, the reference data being associated with respective age ranges and with a given population type.

12. A system according to any preceding claim, the system being configured to enable personal information about the subject to be input.

13. A system according to claim 12, wherein the system is configured to provide one questionnaire, e.g. electronically, enabling said information to be obtained in reply.

14. A system according to any preceding claim, wherein the system is configured to deliver, e.g. on a page that is displayed by the display device, a graphical representation, e.g. a histogram, showing the results of the analysis in comparison with the reference data.

15. A system according to any preceding claim, wherein the system comprises data storage space for storing the results of the analysis.

16. A system according to any preceding claim, the apparatus (15) for collecting sebum on the skin comprising a handle (16) with one end arranged to receive the sebum-collector pellet (17).

17. A system according to any preceding claim, the pellet (17) being packaged prior to use in a strip (19).

18. A system according to any preceding claim, the apparatus (15) for collecting sebum on the skin being constituted by a sebumeter.

19. A system according to claim 1, the camera (4) being used to acquire the image of the collector medium.

## Patentansprüche

1. System (1), das die Analyse der Haut einer Person gestattet, umfassend:
- eine Kamera (4), um mindestens ein Bild der Haut mit einer Vergrößerung zu erfassen, die es gestattet, die Poren und/oder die Flecken der Haut zu beobachten,
- Anzeigemittel (8), um mindestens ein erfasstes Bild und ein Bild einer Bezugshaut gleichzeitig anzuzeigen,
- ein anderes Gerät (12; 13; 15) als eine Kamera, um ein physikalischchemisches Merkmal der Haut zu messen,
- Behandlungsmittel (5), um eine den Zustand der Haut betreffende Information ausgehend von der Verarbeitung mindestens eines erfassten Bildes und/oder einer von dem Gerät durchgeführten Messung zu liefern,
- ein Gerät (15), um auf der Haut Sebum zu sammeln, umfassend einen Sebumsammelträger, **dadurch gekennzeichnet, dass** er ein Plättchen (17) umfasst, das eine Seite zum Auflegen auf die Haut besitzt, und dass die Verarbeitungsmittel (5) ausgebildet sind, um ausgehend von der Verarbeitung eines Bilds des Sammelträgers (17) die gesammelte Sebummenge zu bestimmen.

2. System nach dem vorhergehenden Anspruch, **dadurch gekennzeichnet, dass** die Kamera (4) ein Objektiv umfasst, das mehr als 40-fach, beispielsweise 60-fach vergrößert.

3. System nach einem der Ansprüche 1 und 2, **dadurch gekennzeichnet, dass** die Verarbeitungsmittel (5) ausgebildet sind, um ausgehend von mindestens einem von der Kamera erfassten Bild der Haut mindestens eines der Anzahl, der Größe und der Lokalisierung der Poren der Haut zu bestimmen.

4. System nach einem der vorhergehenden Ansprüche, **dadurch gekennzeichnet, dass** die Verarbeitungsmittel (5) ausgebildet sind, um ausgehend von mindestens einem von der Kamera erfassten Bild der Haut mindestens eines der Anzahl, der Größe und der Lokalisierung der Flecken der Haut zu bestimmen.

5. System nach einem der vorhergehenden Ansprüche, **dadurch gekennzeichnet, dass** die Kamera (4) einen Polarisator umfasst.

6. System nach einem der vorhergehenden Ansprüche, **dadurch gekennzeichnet, dass** es eine zweite Kamera (11) umfasst, die ein Objektiv mit kleinerer Vergrößerung als derjenigen der ersten Kamera, insbesondere mit einem Vergrößerungsfaktor gleich 10 umfasst, um mindestens ein Bild der Haut zu erfassen, das es gestattet, die Falten der Haut zu analysieren.

7. System nach dem vorhergehenden Anspruch, **dadurch gekennzeichnet, dass** die Verarbeitungsmittel (5) ausgebildet sind, um einen Rauheitsgrad der Haut ausgehend von einem Bild der Falten der Haut zu bestimmen.

8. System nach einem der vorhergehenden Ansprüche, **dadurch gekennzeichnet, dass** es ein Elastometer (12) umfasst, um einen Festigkeitsgrad der Haut zu bestimmen.

9. System nach einem der vorhergehenden Ansprüche, **dadurch gekennzeichnet, dass** es ein Corneometer (13) umfasst, um einen Hydratisierungsgrad der Haut zu bestimmen.

10. System nach einem der vorhergehenden Ansprüche, **dadurch gekennzeichnet, dass** die Verarbeitungsmittel (5) ausgebildet sind, um den Vergleich der sich aus der Analyse ergebenden Daten mit Bezugsdaten zu gestatten.

11. System nach dem vorhergehenden Anspruch, **dadurch gekennzeichnet, dass** die Bezugsdaten ausgehend von Messungen an einer Gruppe von Personen erhalten werden, deren Haut als ideal betrachtet wird, wobei die Bezugsdaten jeweiligen Altersgruppen und einem gegebenen Bevölkerungstyp zugeordnet sind.

12. System nach einem der vorhergehenden Ansprüche, **dadurch gekennzeichnet, dass** es ausgebildet ist, um die Eingabe von persönlichen Daten der Person zu gestatten.

13. System nach Anspruch 12, **dadurch gekennzeichnet, dass** es ausgebildet ist, um einen Fragebogen insbesondere in elektronischer Form zu liefern, der es gestattet, als Antwort diese Daten zu erhalten.

14. System nach einem der vorhergehenden Ansprüche, **dadurch gekennzeichnet, dass** es ausgebildet ist, um beispielsweise in der Form einer von den Anzeigemitteln angezeigten Seite eine graphische Darstellung insbesondere vom Typ Histogramm zu liefern, die das Ergebnis der Analyse mit gegenüberstehenden Bezugsdaten anzeigt.

15. System nach einem der vorhergehenden Ansprüche, **dadurch gekennzeichnet, dass** es einen Raum zum Speichern von Daten umfasst, um das Ergebnis der Analyse zu speichern.

16. System nach einem der vorhergehenden Ansprüche, wobei das Gerät (15) zum Sammeln von Sebum auf der Haut ein Greiforgan (16) umfasst, von dem ein Ende ausgebildet ist, um das Plättchen (17) aufzunehmen.

17. System nach einem der vorhergehenden Ansprüche, wobei das Plättchen (17) vor Verwendung in einer Platte (19) verpackt ist.

18. System nach einem der vorhergehenden Ansprüche, wobei das Gerät (15) zum Sammeln von Sebum auf der Haut ein Sebumeter ist.

19. System nach Anspruch 1, wobei das Bild des Sammelträgers mit Hilfe der Kamera (4) erfasst wurde.
